# EUROPEAN PATENT SPECIFICATION

(11) **EP 2 949 282 B1**
(45) Date of publication and mention of the grant of the patent: **13.11.2019**
(21) Application number: 15169673.9
(22) Date of filing: 28.05.2015
(51) Int. Cl.: A61B 18/14, A61M 25/00, A61B 18/00, A61B 17/00

(54) **CATHETER ELECTRODE WITH MULTIPLE THERMOCOUPLES**
KATHETERELEKTRODE MIT MEHREREN THERMOELEMENTEN
ÉLECTRODE DE CATHÉTER AVEC PLUSIEURS THERMOCOUPLES

(30) Priority: 29.05.2014 US 201414289802
(43) Date of publication of application: 02.12.2015
(73) Proprietor: Biosense Webster (Israel) Ltd., 2066717 Yokneam (IL)
(72) Inventor: GOVARI, Assaf, 3440001 Haifa (IL); EPHRATH, Yaron, 3707969 Karkur (IL); BEECKLER, Christopher Thomas, Brea, CA 92821 (US)
(74) Representative: Carpmaels & Ransford LLP

(56) References cited:
- EP-A1- 2 554 134
- US-A- 6 162 184
- US-A1- 2010 057 072
- US-B1- 6 547 788

## Description

### FIELD OF THE INVENTION

The present invention relates generally to medical devices, and particularly to methods and systems for temperature sensing in intra-body medical probes.

### BACKGROUND OF THE INVENTION

Various types of medical probes, such as cardiac ablation catheters, comprise means for sensing temperature. For example, U.S. Patent 5,769,847, describes a system and associated method that ablate body tissue using multiple emitters of ablating energy. The system and method convey ablating energy individually to each emitter in a sequence of power pulses. The system and method periodically sense temperature at each emitter and compare the sensed temperatures to a desired temperature established for all emitters to generate a signal individually for each emitter based upon the comparison. The system and method individually vary the power pulse to each emitter based upon the signal for that emitter to maintain the temperatures at all emitters essentially at the desired temperature during tissue ablation.

U.S. Patent Application Publication 2011/0160716, describes a medical probe including an ablation electrode and a first conductor connected to the ablation electrode. The first conductor is configured to convey ablation energy to the ablation electrode. The probe also includes a second conductor which is connected at a junction to the first conductor so as to form a thermocouple at the junction.

US2010/0057072A1 discloses an ablation catheter for performing tissue ablation, having an elongate shaft with a lumen and a tip ablation electrode at the distal end of the shaft. The tip electrode has a fluid exit port opening through the surface of the tip electrode and at least one channel extending along the surface of the tip electrode to allow fluid passage in the event that the fluid exit port is blocked. The catheter has a connector to a cooling fluid source, and a fluid delivery tube within the shaft lumen to deliver cooling fluid from the connector to the fluid exit port.

US 6,162184 discloses a system for use in catheter-based tissue ablation systems employing thermocouples for temperature sensing.

### SUMMARY OF THE INVENTION

The invention is defined by the independent claims. In the following any aspect, example and embodiment which does not fall under the scope of the independent claims is not part of the invention. An embodiment of the present invention that is described herein provides a medical probe. The probe includes an elongate body for insertion into an organ of a patient, and an electrode that is attached to the elongate body. Multiple thermocouples are disposed at respective different locations of the electrode and electrically coupled to the electrode, and are configured to sense respective temperatures at the locations.

In some embodiments, the probe includes a first conductor that is connected in common to the thermocouples, and multiple second conductors that are each connected to a respective one of the thermocouples, such that electrical potentials between the first conductor and the second conductors are indicative of the respective temperatures of the thermocouples.

In an example embodiment, the first conductor includes copper, and the second conductors include constantan. In a disclosed embodiment, the electrode includes an ablation electrode, and the first conductor or one of the second conductors is also used for delivering Radio Frequency (RF) current to the electrode. In an embodiment, the probe includes electrical connections that electrically-connect the first conductor to the electrode at the respective locations of the thermocouples.

In an embodiment, at least some of the thermocouples are disposed around a perimeter of the electrode. Additionally or alternatively, at least some of the thermocouples are disposed along the electrode, in parallel with a longitudinal axis of the elongate body.

In another embodiment, the electrode includes irrigation holes for delivering irrigation fluid, and the multiple thermocouples are distributed among the irrigation holes. In yet another embodiment, the thermocouples are potted into one or more openings using electrically-conducting potting material. In still another embodiment, the electrode forms a circumferential ring around the elongate body of the medical probe. In an alternative embodiment, the electrode, including the thermocouples, is attached to a balloon.

There is additionally provided, in accordance with an embodiment of the present invention, an electrode including an electrode body and multiple thermocouples. The electrode body is attached to a medical probe inserted into an organ of a patient. The multiple thermocouples are electrically coupled at respective different locations of the electrode body and electrically coupled to the electrode body, and are configured to sense respective temperatures at the locations. In some embodiments the electrode includes an ablation electrode and the electrode body is metallic.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a system for cardiac electrophysiological (EP) mapping and ablation, in accordance with an embodiment of the present invention;
Fig. 2 is a schematic, pictorial illustration of an irrigated tip ablation electrode, in accordance with an embodiment of the present invention;
Fig. 3 is a schematic, pictorial illustration of a ring ablation electrode, in accordance with an embodiment of the present invention; and
Figs. 4 and 5 are diagrams showing interconnection of multiple Thermocouples (TCs) in an ablation electrode, in accordance with an embodiment of the present invention.

### DETAILED DESCRIPTION OF EMBODIMENTS

### OVERVIEW

Embodiments of the present invention that are described herein provide improved medical probes for performing cardiac ablation and other minimally-invasive medical procedures. In the disclosed embodiments, a medical probe such as a cardiac catheter comprises an electrode having multiple thermocouples disposed thereon. The thermocouples are configured to sense the temperature at their respective locations, and thus provide a mapping of the temperature across the electrode.

Measuring temperature at multiple locations on a given electrode is important, for example, in scenarios in which the temperature gradients across the electrode are large. Such gradients occur, for example, in irrigated tip ablation electrodes. Nevertheless, the disclosed techniques are suitable for use with various other types of probes and electrodes.

In some embodiments, the wiring connected to the thermocouples is simplified by sharing one of the thermocouple conductors among multiple thermocouples. In an example embodiment, the thermocouples share a common copper conductor. The second conductor, e.g., a constantan conductor, is separate for each thermocouple. Additionally or alternatively, one of the thermocouple conductors, e.g., a common copper conductor, may also be used for delivering Radio Frequency (RF) current to the electrode.

Specific example implementations of irrigated tip and ring electrodes are described herein. Several techniques for mounting the thermocouples, e.g., using welding or potting with electrically-conductive-epoxy, are proposed.

### SYSTEM DESCRIPTION

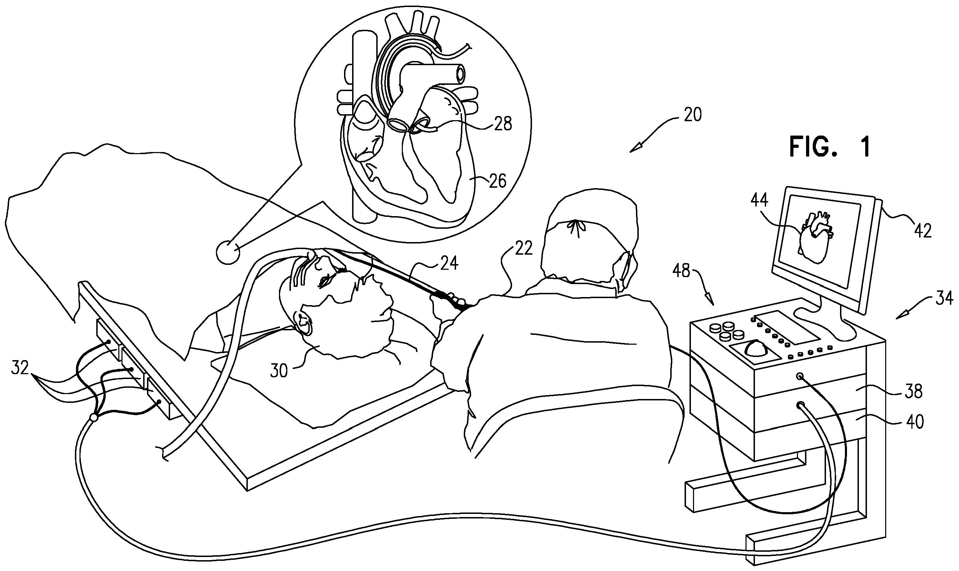

Fig. 1 is a schematic, pictorial illustration of a system 20 for cardiac electrophysiological (EP) mapping and ablation, in accordance with an embodiment of the present invention. System 20 comprises a probe, such as a catheter 24, and a control console 34. In the embodiment described hereinbelow, catheter 24 is used for EP mapping an ablation in a heart 26 of a patient 30. Alternatively, catheter 24 or other suitable probes may be used, *mutatis mutandis*, for other therapeutic purposes in the heart. In one embodiment, system 20 is based on the CARTO™ system produced by Biosense-Webster, Inc. (Diamond Bar, California).

In the example system of Fig. 1, an operator 22, such as a physician, inserts catheter 24 through the vascular system of patient 30 so that a distal end 28 of the catheter enters a chamber (e.g., a ventricle or atrium) of heart 26. Catheter 24 is typically connected by a suitable connector at its proximal end to console 34.

In this embodiment, system 20 uses magnetic position sensing to determine position coordinates of distal end 28 of catheter 24 inside heart 26. For this purpose, a driver circuit 38 in console 34 drives field generators 32 to generate magnetic fields within the body of patient 30. Typically, field generators 32 comprise coils, which are placed below the patient's torso at fixed, known positions. These coils generate magnetic fields in a predefined working volume that contains heart 26. A magnetic field sensor (not shown) within the distal end of catheter 24 generates electrical signals in response to these magnetic fields.

A signal processor 40 processes these signals in order to determine the position coordinates of the distal end of catheter 24, typically including both location and orientation coordinates. This method of position sensing is implemented, for example, in the above-mentioned CARTO system. Alternatively or additionally, system 20 may use other methods of position sensing that are known in the art, such as ultrasonic or electrical impedance-based methods.

Processor 40 in console 34 typically comprises a general-purpose computer processor, with suitable front end and interface circuits for receiving signals from catheter 24 and for controlling and receiving inputs from the other components of console 34. Processor 40 may be programmed in software to carry out the functions that are described herein. The software may be downloaded to processor 40 in electronic form, over a network, for example, or it may be provided, alternatively or additionally, on tangible, non-transitory media, such as optical, magnetic or electronic memory media. Further alternatively or additionally, some or all of the functions of processor 40 may be carried out by dedicated or programmable digital hardware components.

Based on the signals received from catheter 24 and other components of system 20, processor 40 drives a display 42 to present operator 22 with a three-dimensional (3D) map 44 of heart 26. The map may indicate cardiac electrophysiological activity measured by catheter 24, as well as providing visual feedback regarding the position of the catheter in the patient's body and status information and guidance regarding the procedure that is in progress. Operator 22 may control system 20 using various input devices 48 on console 34.

Although Fig. 1 shows a particular system configuration and application environment, the principles of the present invention may similarly be applied in other therapeutic applications using not only catheters, but also medical probes of other types.

### CATHETER ELECTRODES WITH MULTIPLE THERMOCOUPLES

In some embodiments of the present invention, catheter 24 comprises an electrode having multiple thermocouples disposed thereon. The thermocouples are electrically-coupled to the electrode body. The thermocouples are configured for measuring the temperature at multiple respective locations on the electrode. The measured temperatures are then used by processor 40 in console 34 for controlling the procedure as appropriate. In the embodiments described herein the electrode in question is an ablation electrode. Alternatively, however, the disclosed techniques can be used with various other suitable types of electrodes.

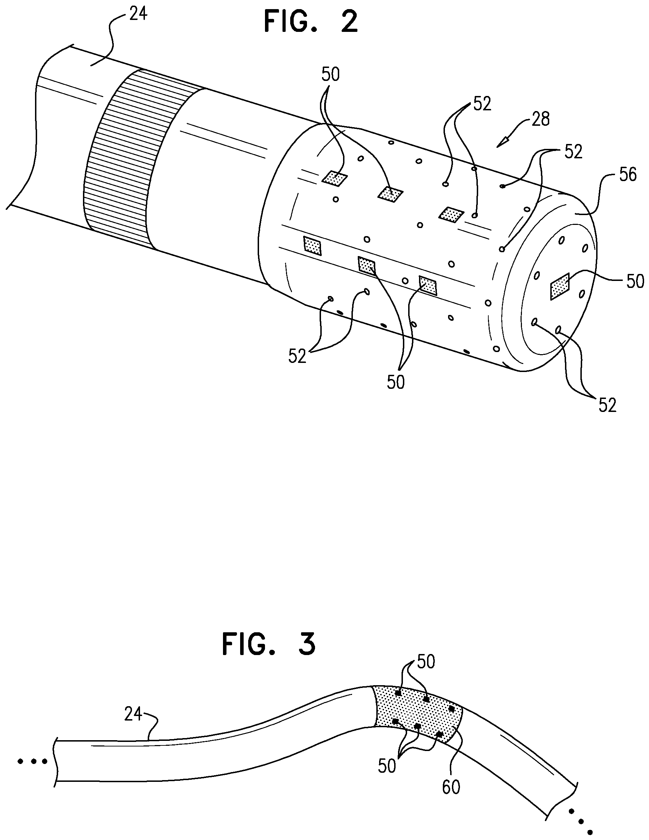

Fig. 2 is a schematic, pictorial illustration of an irrigated tip ablation electrode, in accordance with an embodiment of the present invention. The electrode of Fig. 2 is located at distal end 28 of catheter 24. The electrode comprises a metallic electrode body 56, which is brought into contact with a selected spot on the inner heart surface and delivers Radio-Frequency (RF) current to a selected spot.

Multiple irrigation holes 52 are formed in electrode body 56. Irrigation fluid is pumped through holes 52 in order to cool the electrode and surrounding tissue during the ablation procedure. When using an ablation electrode of this sort, considerable temperature gradients may develop across the electrode. Such gradients may be on the order of 10°C, for example.

In order to monitor and control the temperature during ablation, the electrode of Fig. 2 comprises multiple thermocouples (TCs) 50 that are mounted at multiple respective locations of the electrode, typically distributed among the irrigation holes. In Fig. 2 the TCs are shown on the outside of body 56, for the sake of clarity. Alternatively, however, the TCs may be coupled to the interior of body 56, i.e., below the electrode surface, since metallic body 56 is thermally conductive.

In various embodiments, any suitable number of TCs 50 may be used, and the TCs may be distributed across body 56 in any suitable way. In an example embodiment, at least some of the TCs are distributed around the perimeter of the electrode. Additionally or alternatively, at least some of the TCs may be distributed along the electrode, in parallel with the longitudinal axis of catheter 24.

Fig. 3 is a schematic, pictorial illustration of a ring ablation electrode, in accordance with an alternative embodiment of the present invention. In the present example, the ablation electrode is mounted along a section of catheter 24, not necessarily at the very end of the distal tip. In an example embodiment, the distal tip of the catheter is annular or lasso-shaped, and one or more such ablation electrodes are mounted along the ring- or lasso-shaped distal tip.

In this example, too, the electrode comprises a metallic electrode body 60, and multiple TCs 50 mounted at suitable locations of the electrode. The TCs may be mounted on the exterior or on the interior of body 60. Any suitable number of TCs 50 may be used, and the TCs may be distributed across body 56 in any suitable way (e.g., around the electrode perimeter and/or along the electrode body).

The electrode configurations of Figs. 2 and 3 are chosen purely for the sake of conceptual clarity. In alternative embodiments, any other suitable electrode configuration, and any other suitable configuration of TCs on the electrode, can be used.

### THERMOCOUPLE MOUNTING AND INTERCONNECTION SCHEMES

In various embodiments, thermocouples (TCs) 50 may comprise any suitable type of TC. The description that follows refers mainly to Type T, copper-constantan (Cu-Co) TCs, but any other suitable TC type can be used in alternative embodiments.

In the present example, each TC 50 is formed at the junction of a copper (cu) conductor and a constantan (co) conductor. The electrical potential developing at this junction is indicative of the junction temperature. Processor 40 typically senses the temperature of a given TC by sensing the electrical potential of the TC junction.

In some embodiments, one of the conductors is shared by two or more of TCs 50 (possibly by all the TCs). In an example embodiment, all the TCs share a common copper conductor, while the constantan conductors are separate for each TC. In this configuration, for N TCs, the number of conductors that need to be routed through catheter 24 to console 34 is only N+1 (instead of 2N). This technique simplifies the catheter wiring, enables reduction in catheter diameter, and/or frees internal volume in the catheter for other purposes.

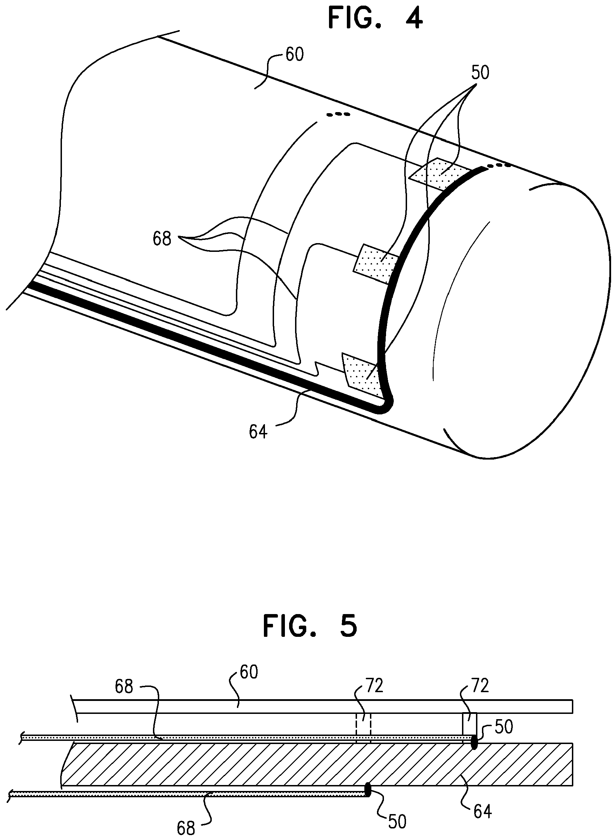

Fig. 4 is a diagram showing interconnection of TCs 50 in an ablation electrode, in accordance with an embodiment of the present invention. In the present example, a copper conductor 64 is connected in series to TCs 50, i.e., is common to multiple TCs. Multiple constantan conductors 68 are connected to TCs 50, respectively, i.e., a separate constantan conductor 68 per TC.

Conductors 64 and 68 may run through catheter 24 to console 34, or they may run through a partial length of the catheter, e.g., as far as the catheter handle. At the far side of the conductors, suitable circuitry senses the electrical potential between copper conductor 64 and each constantan conductor 68. These electrical potentials are indicative of the temperatures of the respective TCs.

In some embodiments, a common conductor (e.g., copper conductor) is shared by all TCs 50 of the electrode. In alternative embodiments, the TCs are divided into groups, and the TCs in each group share a common conductor. Other suitable interconnection schemes can also be used.

Fig. 5 is a cross-section diagram showing interconnection of TCs 50 in an ablation electrode, in accordance with an embodiment of the present invention. In this example, conductor 64 is a 40-gauge copper conductor that is common to multiple TCs 50. Conductors 68 are 48-gauge constantan conductors, one per each TC 50.

In the present example, copper conductor 64 is also electrically connected to electrode body 60, and is used for delivering RF current from a generator in console 34 to electrode body 60. Electrical connection between copper conductor 64 and electrode body 60 is provided by one or more connections 72. As can be seen in the figure, connections 72 are collocated with TCs 50. In other words, copper conductor 64 is electrically-connected to electrode body 60 at the locations of the thermocouples. Typically, the gauge of conductor 64 is chosen to withstand the RF current level of the ablation signal.

The examples of Figs. 4 and 5 refer to the ring electrode of Fig. 3. Alternatively, however, the disclosed interconnection schemes can be used in the irrigated tip electrode of Fig. 2, as well as in any other suitable electrode.

TCs 50 are typically attached to the electrode body using some thermally-conductive means, so that the temperature sensed by the TCs will reflect the actual electrode temperature. Any suitable attachment means can be used, such as welding, bonding or potting. In an example embodiment, TCs 50 are fitted in one or more holes formed in the electrode body, and held in place using a suitable potting material. The potting material may comprise, for example, electrically-conductive epoxy.

In some embodiments, the electrode, including the multiple thermocouples, is attached to a balloon that is inserted into the patient body. In these embodiments, the electrode may comprise, for example, a flexible circuit or flat thin metal stripe that is bonded to an inflatable balloon. The thermocouples may be attached to the front or back of the electrode surface. When the balloon is inflated, at least part of the electrode, and the corresponding thermocouples, make contact with target tissue.

Although the embodiments described herein mainly address cardiac catheters and ablation electrodes, the methods and systems described herein can also be used in various other systems and applications that can benefit from multiple temperature measurements across an electrode.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art.

## Claims

1. A medical probe (24), comprising:
an elongate body for insertion into an organ (26) of a patient;
an electrode comprising an electrode body (56), which is attached to the elongate body of the probe (24);
multiple thermocouples (50), which are disposed at respective different locations of the electrode, and which are configured to sense respective temperatures at the locations; and
a first conductor (64) that is directly connected in series with the thermocouples (50) to provide a conductor that is common to the thermocouples (50), and multiple second conductors (68) that are each connected to a respective one of the thermocouples (50), wherein electrical potentials between the first conductor (64) and the second conductors (68) are indicative of the respective temperatures of the thermocouples;
wherein the electrode comprises an ablation electrode, and **characterized in that** the thermocouples (50) are electrically coupled to the electrode, and the first conductor (64) or one of the second conductors (68) is also configured to be used for delivering Radio Frequency (RF) current to the electrode body (56).

2. The probe (24) according to claim 1, wherein at least some of the thermocouples (50) are disposed around a perimeter of the electrode, or are disposed along the electrode, in parallel with a longitudinal axis of the elongate body.

3. The probe (24) according to claim 1, wherein the electrode comprises irrigation holes (52) for delivering irrigation fluid, and wherein the multiple thermocouples (50) are distributed among the irrigation holes (52).

4. The probe (24) according to claim 1, wherein the thermocouples (50) are potted into one or more openings using electrically-conducting potting material.

5. The probe (24) according to claim 1, wherein the electrode forms a circumferential ring around the elongate body of the medical probe (24).

6. The probe (24) according to claim 1, wherein the electrode, including the thermocouples (50), is attached to a balloon.

7. An electrode, comprising:
an electrode body (56), which is attached to a medical probe (24) insertable into an organ (26) of a patient;
multiple thermocouples (50), which are electrically coupled at respective different locations of the electrode body (56) and which are configured to sense respective temperatures at the locations; and
a first conductor (64) that is directly connected in series with the thermocouples (50) to provide a conductor that is common to the thermocouples (50), and multiple second conductors (68) that are each connected to a respective one of the thermocouples (50), wherein electrical potentials between the first conductor (64) and the second conductors (68) are indicative of the respective temperatures of the thermocouples (50);
wherein the electrode comprises an ablation electrode, and **characterized in that** the first conductor (64) or one of the second conductors (68) is also configured to be used for delivering Radio Frequency (RF) current to the electrode body (56).

8. The electrode according to claim 7, wherein the electrode body (56) is metallic.

9. The probe (24) according to claim 1 or the electrode according to claim 7, wherein the first conductor (64) comprises copper, and wherein the second conductors (68) comprise constantan.

10. The probe (24) according to claim 1 or the electrode according to claim 7, and comprising electrical connections that electrically-connect the first conductor (64) to the electrode body (56) at the respective locations of the thermocouples (50).

11. The electrode according to claim 7, wherein at least some of the thermocouples (50) are disposed around a perimeter of the electrode body (56), or are disposed along the electrode body (56), in parallel with a longitudinal axis of the medical probe (24).

12. The electrode according to claim 7, wherein the electrode body (56) comprises irrigation holes for delivering irrigation fluid, and wherein the multiple thermocouples (50) are distributed among the irrigation holes.

13. The electrode according to claim 7, wherein the thermocouples (50) are potted into one or more openings in the electrode body (56) using electrically-conducting potting material.

14. The electrode according to claim 7, wherein the electrode forms a circumferential ring around an elongate body of the medical probe (24).

15. The electrode according to claim 7, wherein the electrode, including the thermocouples (50), is attached to a balloon.

## Patentansprüche

1. Medizinische Sonde (24), umfassend:
einen länglichen Körper zur Einführung in ein Organ (26) eines Patienten;
eine Elektrode, umfassend einen Elektrodenkörper (56), der am länglichen Körper der Sonde (24) befestigt ist;
mehrere Thermoelemente (50), die an jeweiligen unterschiedlichen Stellen der Elektrode angeordnet sind, und die zum Erfassen jeweiliger Temperaturen an den Stellen ausgelegt sind; und
einen ersten Leiter (64), der mit den Thermoelementen (50) direkt in Reihe verbunden ist, um einen Leiter bereitzustellen, der den Thermoelementen (50) gemeinsam ist, und mehrere zweite Leiter (68), die jeweils mit einem jeweiligen der Thermoelemente (50) verbunden sind, wobei elektrische Potentiale zwischen dem ersten Leiter (64) und den zweiten Leitern (68) auf die jeweiligen Temperaturen der Thermoelemente hinweisen;
wobei die Elektrode eine Ablationselektrode umfasst, und **dadurch gekennzeichnet, dass** die Thermoelemente (50) elektrisch mit der Elektrode gekoppelt sind, und der erste Leiter (64) oder einer der zweiten Leiter (68) auch dazu ausgelegt ist, zur Abgabe eines Hochfrequenz- (HF-) Stroms an den Elektrodenkörper (56) verwendet zu werden.

2. Sonde (24) nach Anspruch 1, wobei zumindest einige der Thermoelemente (50) um einen Umfang der Elektrode angeordnet sind oder entlang der Elektrode parallel zu einer Längsachse des länglichen Körpers angeordnet sind.

3. Sonde (24) nach Anspruch 1, wobei die Elektrode Spüllöcher (52) zur Abgabe von Spülflüssigkeit umfasst und wobei die mehreren Thermoelemente (50) unter den Spüllöchern (52) aufgeteilt sind.

4. Sonde (24) nach Anspruch 1, wobei die Thermoelemente (50) mit einem elektrisch leitfähigen Vergussmaterial in die eine oder mehreren Öffnungen eingegossen sind.

5. Sonde (24) nach Anspruch 1, wobei die Elektrode einen Umfangsring um den länglichen Körper der medizinischen Sonde (24) bildet.

6. Sonde (24) nach Anspruch 1, wobei die Elektrode, einschließlich der Thermoelemente (50), an einem Ballon befestigt ist.

7. Elektrode, umfassend:
einen Elektrodenkörper (56), der an einer medizinischen Sonde (24) befestigt ist, die in ein Organ (26) eines Patienten einführbar ist;
mehrere Thermoelemente (50), die an jeweiligen unterschiedlichen Stellen des Elektrodenkörpers (56) elektrisch gekoppelt sind, und die zum Erfassen jeweiliger Temperaturen an den Stellen ausgelegt sind; und
einen ersten Leiter (64), der mit den Thermoelementen (50) direkt in Reihe verbunden ist, um einen Leiter bereitzustellen, der den Thermoelementen (50) gemeinsam ist, und mehrere zweite Leiter (68), die jeweils mit einem jeweiligen der Thermoelemente (50) verbunden sind, wobei elektrische Potentiale zwischen dem ersten Leiter (64) und den zweiten Leitern (68) auf die jeweiligen Temperaturen der Thermoelemente (50) hinweisen;
wobei die Elektrode eine Ablationselektrode umfasst, und **dadurch gekennzeichnet, dass**
der erste Leiter (64) oder einer der zweiten Leiter (68) auch dazu ausgelegt ist, zur Abgabe von Hochfrequenz- (HF-) Strom an den Elektrodenkörper (56) verwendet zu werden.

8. Elektrode nach Anspruch 7, wobei der Elektrodenkörper (56) aus Metall besteht.

9. Sonde (24) nach Anspruch 1 oder Elektrode nach Anspruch 7, wobei der erste Leiter (64) Kupfer umfasst und wobei die zweiten Leiter (68) Konstantan umfassen.

10. Sonde (24) nach Anspruch 1 oder Elektrode nach Anspruch 7, und umfassend elektrische Anschlüsse, die den ersten Leiter (64) elektrisch mit dem Elektrodenkörper (56) an den jeweiligen Stellen der Thermoelemente (50) verbinden.

11. Elektrode nach Anspruch 7, wobei zumindest einige der Thermoelemente (50) um einen Umfang des Elektrodenkörpers (56) angeordnet sind oder entlang des Elektrodenkörpers (56) parallel zu einer Längsachse der medizinischen Sonde (24) angeordnet sind.

12. Elektrode nach Anspruch 7, wobei der Elektrodenkörper (56) Spüllöcher zur Abgabe von Spülflüssigkeit umfasst und wobei die mehreren Thermoelemente (50) unter den Spüllöchern aufgeteilt sind.

13. Elektrode nach Anspruch 7, wobei die Thermoelemente (50) mit einem elektrisch leitfähigen Vergussmaterial in die eine oder mehreren Öffnungen im Elektrodenkörper (56) eingegossen sind.

14. Elektrode nach Anspruch 7, wobei die Elektrode einen Umfangsring um einen länglichen Körper der medizinischen Sonde (24) bildet.

15. Elektrode nach Anspruch 7, wobei die Elektrode, einschließlich der Thermoelemente (50), an einem Ballon befestigt ist.

## Revendications

1. Sonde médicale (24), comprenant :
un corps allongé pour une introduction dans un organe (26) d'un patient ;
une électrode comprenant un corps (56) d'électrode, qui est fixé au corps allongé de la sonde (24) ;
de multiples thermocouples (50), qui sont disposés au niveau de différents emplacements de l'électrode, et qui sont conçus pour détecter des températures respectives au niveau des emplacements ; et
un premier conducteur (64) qui est directement connecté en série avec les thermocouples (50) pour former un conducteur qui est commun aux thermocouples (50), et de multiples seconds conducteurs (68) qui sont chacun connectés à un thermocouple respectif des thermocouples (50), dans lequel les potentiels électriques entre le premier conducteur (64) et les seconds conducteurs (68) sont révélateurs des températures respectives des thermocouples ;
dans laquelle l'électrode comprend une électrode d'ablation, et **caractérisée en ce que** les thermocouples (50) sont couplés électriquement à l'électrode, et le premier conducteur (64) ou un des seconds conducteurs (68) est également conçu pour servir à distribuer un courant radiofréquence (RF) au corps (56) d'électrode.

2. Sonde (24) selon la revendication 1, dans laquelle au moins certains des thermocouples (50) sont disposés autour d'un périmètre de l'électrode, ou sont disposés le long de l'électrode, parallèlement à un axe longitudinal du composant allongé.

3. Sonde (24) selon la revendication 1, dans laquelle l'électrode comprend des trous d'irrigation (52) pour distribuer un fluide d'irrigation, et dans laquelle les multiples thermocouples (50) sont répartis parmi les trous d'irrigation (52).

4. Sonde (24) selon la revendication 1, dans laquelle les thermocouples (50) sont enrobés dans une ou plusieurs ouvertures à l'aide d'un matériau d'enrobage électroconducteur.

5. Sonde (24) selon la revendication 1, dans laquelle l'électrode forme un anneau circonférentiel autour du corps allongé de la sonde médicale (24).

6. Sonde (24) selon la revendication 1, dans laquelle l'électrode, comprenant les thermocouples (50), est fixée à un ballonnet.

7. Électrode, comprenant :
un corps (56) d'électrodes, qui est fixé à une sonde médicale (24) pouvant être introduite dans un organe (26) d'un patient ;
de multiples thermocouples (50), qui sont couplés électriquement au niveau de différents emplacements du corps (56) d'électrode et qui sont conçus pour détecter des températures respectives au niveau des emplacements ; et
un premier conducteur (64) qui est directement connecté en série avec les thermocouples (50) pour former un conducteur qui est commun aux thermocouples (50), et de multiples seconds conducteurs (68) qui sont chacun connectés à un thermocouple respectif des thermocouples (50), dans lequel les potentiels électriques entre le premier conducteur (64) et les seconds conducteurs (68) sont révélateurs des températures respectives des thermocouples (50) ;
l'électrode comprenant une électrode d'ablation, et étant **caractérisée en ce que**
le premier conducteur (64) ou un des seconds conducteurs (68) est également conçu pour servir à distribuer un courant radiofréquence (RF) au corps (56) d'électrode.

8. Électrode selon la revendication 7, dans laquelle le corps (56) d'électrode est métallique.

9. Sonde (24) selon la revendication 1 ou électrode selon la revendication 7, dans laquelle le premier conducteur (64) comprend du cuivre, et dans laquelle les seconds conducteurs (68) comprennent du constantan.

10. Sonde (24) selon la revendication 1 ou électrode selon la revendication 7, et comprenant des connexions électriques qui connectent électriquement le premier conducteur (64) au corps (56) d'électrode au niveau des emplacements respectifs des thermocouples (50).

11. Électrode selon la revendication 7, dans laquelle au moins certains des thermocouples (50) sont disposés autour d'un périmètre du corps (56) d'électrode, ou sont disposés le long du corps (56) d'électrode, parallèlement à un axe longitudinal de la sonde médicale (24).

12. Électrode selon la revendication 7, dans laquelle le corps (56) d'électrode comprend des trous d'irrigation pour distribuer un fluide d'irrigation, et dans laquelle les multiples thermocouples (50) sont répartis parmi les trous d'irrigation.

13. Électrode selon la revendication 7, dans laquelle les thermocouples (50) sont enrobés dans une ou plusieurs ouvertures dans le corps (56) d'électrode à l'aide d'un matériau d'enrobage électroconducteur.

14. Électrode selon la revendication 7, dans laquelle l'électrode forme un anneau circonférentiel autour d'un corps allongé de la sonde médicale (24).

15. Électrode selon la revendication 7, l'électrode, comprenant les thermocouples (50), étant fixée à un ballonnet.
